Europäisches Patentamt

(19) European Patent Office

Office européen des brevets

(11) Publication number: **0 106 501**
**B1**

(12) **EUROPEAN PATENT SPECIFICATION**

(45) Date of publication of patent specification: **17.02.88**

(51) Int. Cl.⁴: **A 61 F 2/08**

(21) Application number: **83305230.1**

(22) Date of filing: **08.09.83**

(54) A synthetic prosthesis for replacement or repair of ligaments or tendons.

(30) Priority: **10.09.82 US 416565**
**10.09.82 US 416600**

(43) Date of publication of application:
**25.04.84 Bulletin 84/17**

(45) Publication of the grant of the patent:
**17.02.88 Bulletin 88/07**

(84) Designated Contracting States:
**AT BE CH DE FR GB IT LI LU NL SE**

(56) References cited:
**EP-A-0 051 954**
**DE-A-2 947 743**
**US-A-3 176 316**
**US-A-4 149 277**
**US-A-4 187 390**
**US-A-4 208 745**

(73) Proprietor: **W.L. GORE & ASSOCIATES, INC.**
**555 Paper Mill Road P.O. Box 9329**
**Newark Delaware 19711 (US)**

(72) Inventor: **Bain, James Raymond**
**1004 West Hazel Way**
**Flagstaff Arizona 86001 (US)**
Inventor: **Bolton, Carl William**
**2809 N. Fremont Boulevard**
**Flagstaff Arizona 86001 (US)**
Inventor: **Bruchman, William Carl**
**1670 E. Appalachian Road**
**Flagstaff Arizona 86001 (US)**

(74) Representative: **Taylor, Derek George et al**
**MATHISEN, MACARA & CO. The Coach House**
**6-8 Swakeleys Road**
**Ickenham Uxbridge UB10 8BZ (GB)**

EP 0 106 501 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The inventive article described herein is a synthetic prosthesis for replacement or repair of ligaments or tendons.

The generally accepted method of repair of ligaments and tendons is through the use of tissue transplanted to the defect site from elsewhere in the body. This method of repair often fails due to a number of factors, including insufficient strength to the transplanted tissues, dependence of the transplanted tissue on revascularization for viability, and inadequate strength of attachment or fixation of the transplanted tissue.

A great need exists for a prosthetic device to replace damaged ligaments and tendons, and there have been a number of previous attempts at providing such devices. However, there is no prosthesis today which is widely accepted. Among the reasons for failure of prosthetic devices are inadequate tensile strength, lack of adequate fixation, deterioration of the device due to mechanical stresses, and deterioration of the prosthesis/tissue interface.

It is known from EP-A-O 051 954 to provide a tensile load-bearing tissue prosthesis for connection between tensile force-applying tissues. Although this prior-art prosthesis has superficial similarity to the prosthesis of the present invention in that both include a plurality of longitudinally adjacent parallel non-adhering strands, which strands can be of polytetrafluoroethylene, the portion of the prior-art prosthesis between its looped ends is enclosed within a tubular sheath which thus isolates the strands from surrounding tissue. This is in direct contrast to the teaching of the present invention.

According to the present invention there is provided in the portion wherein attachment to the tensile force-applying tissue is required, a plurality of longitudinally adjacent parallel non-adhering strands of microporous micpolytetrafluoroethylene material having interconnecting voids defined by nodes and fibrils, the said strands being of a type which permits tissue ingrowth to occur across substantially the entire strand thickness.

It is well known that the applicants, W. L. Gore & Associates, Inc. market a polytetrafluoroethylene vascular graft material under the trade mark GORE-TEX having interconnected voids defined by nodes and fibrils, and which permit substantial tissue ingrowth to occur. Such a material is referred to in EP-A-0 051 954 for use in manufacture of the sheath which is then treated to prevent tissue ingrowth.

It is also known such as from US-A-4 208 745, to provide a vascular prosthesis in the form of a tubing of porous polytetrafluoroethylene having a fibrous structure of nodes and fibrils connecting the nodes together, intended to form an artificial blood vessel. The prior specification indicates a range of preferred pore sizes and tubing wall thicknesses which exhibit a high patency rate. It will accordingly be apparent to those skilled in the art from the teaching of the prior art and of the present specification how to select microporous polytetrafluoroethylene material whose strands are of a type which will permit the necessary tissue ingrowth.

Preferably, the microporous PTFE material is expanded PTFE having a characteristic interstitial dimension of greater than about 7 microns, and a matrix tensile strength of greater than about 20,000 psi (138 MPa) and with a porosity greater than 30%.

It is further preferred that the parallel strands are formed from a plurality of concentric loops of a continuous filament of the material, the loops being elongated in the tensile load direction, and wherein the ends of the plurality of elongated loops are gathered and bonded together to form at least one eyelet for attaching the article to tensile force-applying bone tissue.

Preferred embodiments of this invention permit maximization of tissue attachment strength to the prosthesis without sacrificing prosthetic strength, and when used as a prosthetic ligament provide an initial means of fixation of the device which allows the patient nearly immediate mobility. This reduced interval of immobility has the effect of greatly reducing required rehabilitation time for the affected joined.

This invention will now be particularly described, by way of example only, by reference to the accompanying drawings in which:—

Figure 1 is a photomicrograph of the PTFE material used in the construction of the prosthesis of Example B;

Figure 2 is a photomicrograph of the PTFE material used in the construction of another prosthesis;

Figure 3 is a photomicrograph of the material of Figure 2 laterally stretched to provided for measurement of characteristic interstitial dimensions;

Figure 4 shows a schematic perspective view of one prosthesis constructed in accordance with the present invention;

Figure 5 depicts schematically the implantation of an anterior cruciate ligament prosthesis not constructed in accordance with the present invention;

Figure 6 depicts schematically a stage in one method of construction of a prosthesis of the present invention;

Figure 7 shows a schematic perspective view of another prosthesis constructed in accordance with the present invention;

Figures 8A, B and C depict the implantation of the prosthesis of Figure 7 into a knee joint as an anterior cruciate ligament prosthesis.

The inventive article described herein is a synthetic prosthesis for replacement or repair of ligaments or tendons. The prosthesis is made up of multiple strands of porous PTFE. The porosity of the strands is characterized by interconnecting void space throughout.

Strand dimensions are small enough to permit tissue growth in and through the entire strand. The percent void space, or porosity, is greater than 30%, which allows mechanical attachment to tissue in the interstitial spaces of the prosthesis to provide sufficient attachment strength. This degree of porosity is a requirement only for those sections of the device which are intended to be anchored through tissue fixation. Porosity, as used here is defined as;

$$\% \text{ Porosity} = \left[1 - \frac{\rho_2}{\rho_1}\right] 100$$

where: $\rho_2$ = density of porous material
$\rho_1$ = density of solid PTFE making up the solid content of the porous material. For PFTE which has never been sintered $\rho_1$ = 2.3 gm/cm$^3$ and for materials which have been sintered a value of 2.2 gm/cm$^3$ is used for $\rho_1$, although this value can actually vary somewhat depending on sintering and cooling conditions.

Immediate postoperative attachment of the device is provided by eyelets which are attached directly to bony tissue. This intitial attachment is augmented and finally made redundant as tissue grows into the porous strand material, providing permanent attachment of the prosthesis to tissue. Tissue can easily grow between and among the strands since they are not attached to each other nor held together tightly. However, the depth to which tissue can grow into each strand is governed by the dimensions of the interconnected void corridors or pathways through the porous microstructure. The complex intercommunicating void space is formed by the solid PTFE matrix. In some cases, the matrix is made up of large solid nodes interconnected by long flexible, relatively inelastic fibrils. Although the nodes may present rigid inflexible structures to ingrowing tissue, the fibrils can be bent and pushed aside by penetrating tissue. Other microstructures of this invention have much smaller nodes which appear merely as connection points for the fibrils. In both cases, the strength of the fibrils in tension is very high, and although they can be bent by tissue, they cannot be stretched significantly. The microstructures of this invention can be characterized by a mean interstitial dimension which can be used to predict the depth of tissue ingrowth. Short fibril lengths impede and bottleneck tissue invasion. Thus, for porous strands having short fibril lengths, the overall strand dimensions must itself be small enough so that ingrowth and attachment will occur throughout the entire strand.

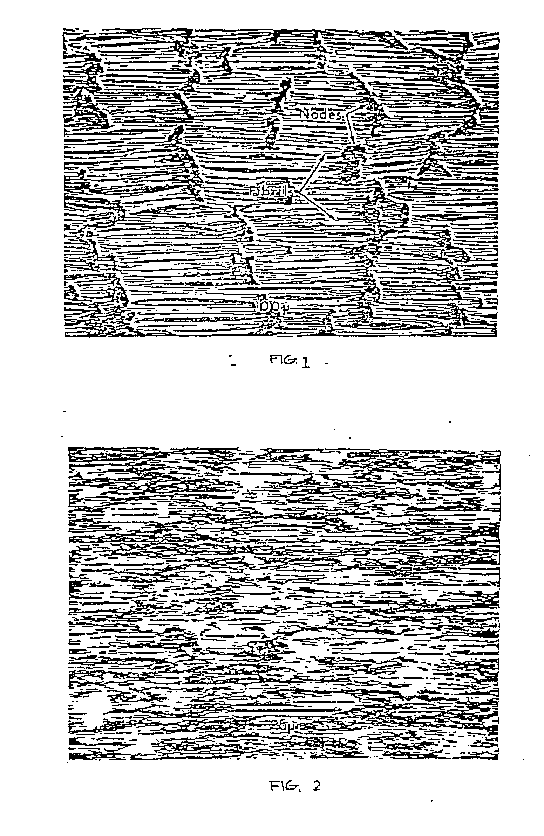

The methods used here to characterize the fibril length of a particular microstructure rely on visual examination of that microstructure. Photographs at a convenient magnification can be provided through scanning electron microscopy or, in some cases, light microscopy. The microporous PTFE materials of this invention can vary sufficiently in their microstructure to that different techniques of measuring the characteristic interstitial dimension must be used. Strand fibres such as those made by the process described in Example B possess a microstructure which can clearly be characterized by nodes interconnected by fibrils. The characteristic interstitial dimension for materials of this tybpe can be determined through a direct measurement of the spacing between nodes. This measurement is taken along a line placed in the direction of strength orientation (Figure 1). A large enough number of measurements must be taken so that the node spacing is adequately characterized. The mean node spacing thus provided is used to characterize the interstitial space and thereby predict the depth of ingrowth into that microstructure.

In strand material which has been manufactured by a stretching process such as is described in U.S. Pat. No. 3,962,153, or the products of U.S. Pat. No. 4,187,390, the nodes of PTFE can be smaller and much less defined. In highly stretched products made according to these patents, node spacing becomes very large and fibrils are packed together. The sintering step in production of these materials causes the bundles of fibrils to coalesce and form secondary attachment points. For this reason, the microstructure of such materials is not readily apparent even under magnification. In determining the characteristic interstitial dimension of these materials, it is necessary to measure the distance between fibril suspension points rather than measuring the fibril length (i.e. node spacing). The interstitial dimensions of these materials can be observed if samples are prepared for microscopy by slightly stretching the material at right angles to its direction of strength orientation. Upon stretching the sample 10% in the lateral direction, with the sample restrained from shrinking in the longitudinal direction, the points at which fibrils are connected become apparent under microscopic examination. The distance between fibril connections is then measured at all obvious gaps created between fibril bundles. This measurement is taken in the direction of strength orientation. As with the method described previously for node spacing, the number of measurements of fibril suspension distance must be sufficient to characterize interstitial dimensions of the microstructure.

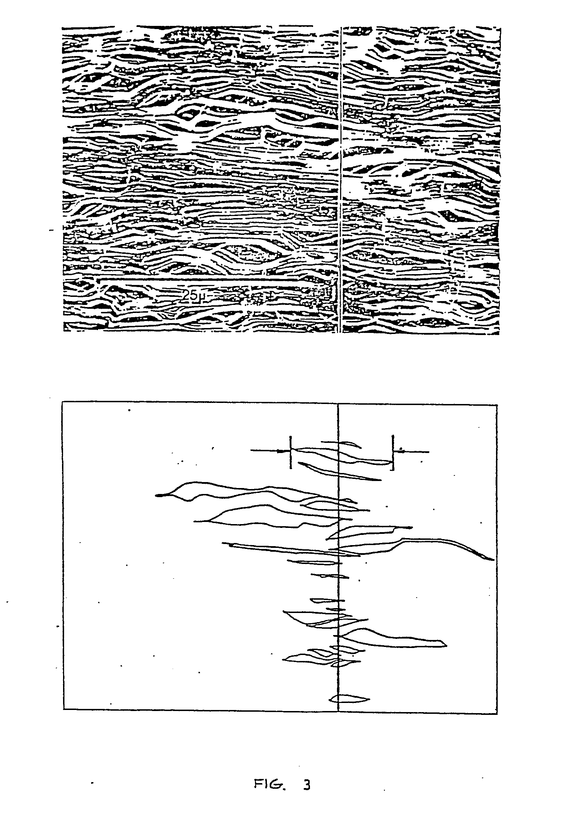

Figure 2 shows how material of this type appears without lateral stretching as compared to Figure 3 which is a micrograph of the same material with 10% lateral stretching. This lateral stretching, which is used only to characterize the microstructure of the material, represents a temporary structural reorientation. A force placed on the material in the longitudinal direction causes a return to the original lateral dimension and restoration of the original microstructure. As previously described, it is believed that the fibrils composing this microstructure are pushed aside by ingrowing tissue. The method of measuring the characteristic interstitial dimension for materials of this type is shown in Figure 3. Having once determined the characteristic interstitial dimension by the techniques described, the proper strand dimensions can be determined.

The maximum thickness which will allow tissue penetration through the entire strand is approximately two times the tissue penetration depth. Thickness, as used here, refers to the appropriate minor cross-sectional dimension of a strand, e.g. the diameter of a strand of circular cross-section, the thickness of a strand of rectangular cross-section. In general, combinations of characteristic interstitial void dimension and strand thickness which are preferred because they allow complete tissue penetration across the strand cross-section in a shorter time interval, may be determined from the following relationship.

$1n$ (stand diameter) $\leq 2.28 \times 10^{-2}$ (CID) — 4.36
for CID > 7 microns, $\leq 120$ microns
$1n$ (strand diameter) $\leq 6.98 \times 10^{-2}$ (CID) — 9.94
for CID $\leq 120$ microns
where: CID = characteristic interstitial dimension (microns)
$1n$ = natural logarithm
Strand diameter is expressed in inches

The depth of tissue penetration into the microporous structure decreases radically as characteristic interstitial dimension falls below 10 microns. This decrease is due to the fact that in structures with this characteristic spacing and below, only a small number of the interstitial pathways are large enough to admit a single cell of the desired type. At characteristic interstitial dimensions of 120 microns and greater, substantial vascularization accompanies tissue growth and allows for a greatly increased depth of penetration. We believe that this creates an increase in the relation of interstitial dimension and depth of tissue penetration.

A major requirement for a successful ligament or tendon prosthesis is that of adequate strength. In many situations prosthetic materials used to replace these natural structures are subjected to very high tensile loads. The strength of the prosthesis must in some cases be many times that of the peak load to which it will be exposed to compensate for the mechanical properties of the prosthesis which are time-dependent.

From a mechanical strength standpoint, one of ordinary skill in the art would realize that the number of individual strands needed for a particular application will depend on several factors. These include: the individual strand cross-sectional area; and the tensile strength of the individual strand; and the tensile force requirement for that particular application, including any safety factors for creep strain limitations. The individual strands used in this invention can be constructed using the processes described in U.S. Pat. No. 3,953,566, U.S. Pat. No. 3,962,153 or following Example B. It is desirable to use a high matrix tensile strength material in order to minimize the overall physical dimensions of the device and thereby minimize the size of drill holes placed in the bone to mount the device. Matrix tensile strength refers to the strength of the polymer in a porous specimen and is used as defined in U.S. Pat. No. 3,953,566.

In the preferred form of this invention:

The strand material is porous PTFE with a matrix tensile strength greater than 20,000 psi (138MPa), a porosity greater than 30%, and a microstructure characterized by intercommunicating pathways formed by the boundaries of nodes and fibrils.

Strand dimensions and characteristic interstitial dimensions of the microstructure are selected such that tissue ingrowth throughout the strand takes place in a rapid fashion.

Each strand and the finished construction possess sufficient strength necessary to meet the mechanical ·requirements of the particular application.

The parallel strands result from multiple loops formed from a continuous filament of the strand material.

The ends of the multiple loops are gathered and formed into at least one eyelet for attaching the article to bone tissue.

The uniformity of the strand loading of the prosthesis under tensile force is enhanced through:

1. Minimizing differences in loop length used to form the parallel strands.

2. Compression of the loop strands in the eyelet segments at an elevated temperature sufficient to coalesce the material and provide strand-to-strand adhesion.

The prosthesis also includes means for distributing the tensile load among the strands as it passes around a radius, said means including:

1. A twist in the strand bundle about its longitudinal axis.

2. A loose strand braid.

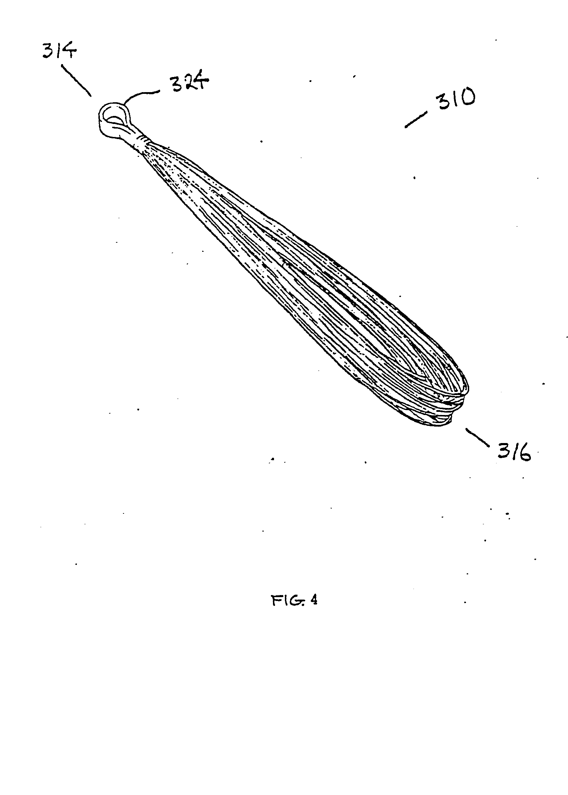

Although the ligament prosthesis embodiment preferably has a pair of opposing eyelets formed form elongated loops, the present invention also encompasses a single eyelet 324 formed in the loops gathered for attachment to bone. The loops at the other end 316 remain ungathered or splayed to provide attachment to soft tissue such as muscle tissue, as by suturing (see Figure 4). In this latter case, the closed loop ends provide additional resistance to possible strand slippage past the sutures. The single eyelet embodiment of this prosthesis 310, could find use in the repair or replacement of tendons.

## Example A

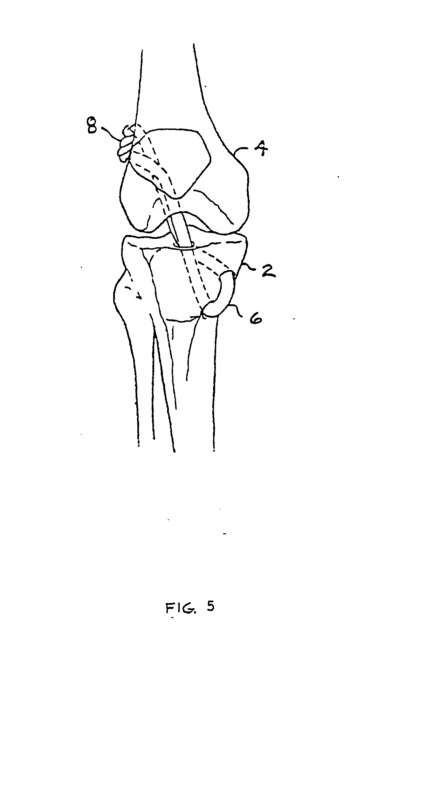

This example demonstrates a prosthetic device which did not achieve satisfactory system strength because the strand thickness was too large for the interstitial dimension which characterized its microstructure. The strand thickness (diameter) was 0.26 inches (0.66 cms), porosity of the strand was approximately 80%, and the characteristic interstitial dimension was about 78 microns. This interstitial dimension was determined as shown in Figure 1. The prosthesis was used to replace the anterior cruciate ligament of a dog by routing the material through drill holes in the tibia and femur. Four holes were drilled in the tibia 2 and femur 4 such that the prosthesis strand 6 formed a loop of material with two strands in the position of the original ligament (Figure 5). Initial fixation was provided by tying the ends of the strand together in a knot 8 to form a continuous loop. Ingrowth and formation of tissue within the interstices of the microporous material were expected to augment the initial fixation strength and to distribute stresses to the surrounding tissue. Each of the strands crossing the knee joint possessed a tensile strength of about 550 pounds (250 kg). The combined strength of these two strands was then 1,100 pounds (499 kg). After having been implanted for 260 days, the knee joint was explanted.

Drill holes were placed in the tibia and femur for mounting into tensile test clamps. After removal of all supporting collateral structures about the knee, the femur was distracted from the tibia along the axis of the prosthetic ligament at a constant rate of 500mm per minute until failure. The length spanning the intra-articular space between bone tunnels represented that portion of the prosthesis placed under tensile load during the test, due to tissue attachment to the prosthesis in the bone tunnels. The failure mode of the system was rupture of the prosthetic device at the level of exit from the bone tunnels. Surprisingly, this rupture took place at a value of only 200 lbs (91 kg). Through histological inspection, we discovered that this reduction in strength was related to the restriction of bony ingrowth to generally less than 1mm depth into the prosthesis. With a strand of this diameter and characteristic interstitial dimension, attachment takes place only at a circumferential ring of material on the periphery of the device. This reduced area then becomes the only load-bearing material of the prosthesis as a tensile force is initially applied. Failure occurs in this circumferential ring of material first and then progresses through the central portion of the prosthesis.

Example B

The experience cited in Example A led to the discovery that tissue ingrowth must penetrate throughout the cross-section of the strand in order to provide adequate long-term system strength. Accordingly, a device was constructed using a strand of similar porosity and characteristic interstitial dimension but with a much smaller diameter. The strand material used to construct the anterior cruciate ligament prosthesis of this example was made as follows:

PTFE dispersion powder ("Fluon CD 123" resin produced by ICI America) was blended with 130cc of "ISOPAR K" odorless solvent (produced by Exxon Corporation) per pound (0.45 kg) of PTFE, compressed into a pellet, and extruded into a 0.108 inch (0.274 cm) diameter rod in a ram extruder having a 96:1 reduction ratio in a cross-section from the pellet to the extruded rod.

The extruded rod still containing Isopar K was immersed in a container of Isopar K at 60°C and stretched to 8.7 times its original length between capstans with an output velocity of about 86.4 ft/min (26.3m/min). These capstans were about 2.8 inches (7.1 cms) in diameter with a center-to-center distance of about 4.5 inches (11.4 cms). The diameter of the rod was reduced from about 0.108 inches (0.274 cm) to about 0.047 inches (0.119 cm) by this stretching. The Isopar K was then removed from this stretched material.

The stretched rod was then pulled through a circular densification die heated to 300°C. The opening in the die tapered at a 10° angle from about 0.050 inches (0.127 cm) to 0.025 inches (0.064 cm) and then was constant for about 0.025 inch (0.064 cm) length. The output velocity of the material exiting the die was 7.2 ft/min (2.19 m/min).

The stretched rod was then heated to 300°C through contact with heated driven capstans and stretched 4 1/2 fold (350%) with an output velocity of 6.5 ft/min (1.98m/min). These capstans had a diameter of 2.75 inches (7 cms) and a center-to-center distance of 4.5 inches (11.4 cms).

Finally the rod was restrained from shrinking and exposed to about 367°C in an air oven for 30 seconds.

In the finished form the fibre made with this process possessed the following characteristics:

Diameter = 0.026 inch (0.066 cm)
Matrix Tensile Strength = 74,000 psi (511 MPa)
Porosity = 80.8%
Characteristics Interstitial Dimension = 74 microns.

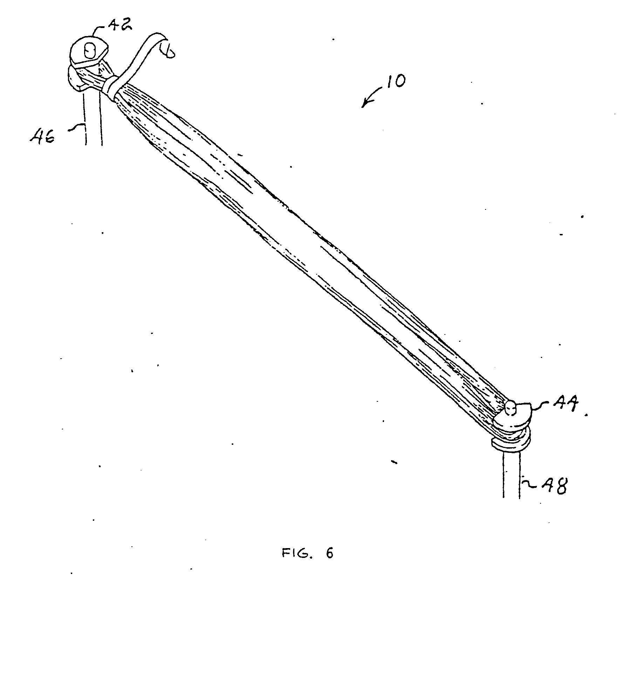

As illustrated in Figure 6, prosthesis 10 was constructed on two steel spools 42,44 which were mounted on a rack (not shown). The spools were supported on studs 46,48 spaced 14cm from center line to center line. These steel spools were threaded to allow demounting of one flange. The strand of PTFE material was passed around these two spools 80 times so that a total of 160 strands connected the two spools. The two free ends of the fibre were tied together with multiple square knots. One spool was demounted from the stud, rotated through 180° and remounted on the stud, thus imparting a one-half twist about the longitudinal axis of the construction. The construction was then wrapped with a thin film of PTFE a total of 25 revolutions each at three locations. This film was manufactured according to the teachings of U.S. Pat. No. 3,962,153 and had the following characteristics:

Width = 0.375 inch (0.95 cm)
Thickness = 0.00025 inch (0.00064 cm)
Longitudinal matrix tensile strength = 70,000 psi (483 MPa)
Porosity = 84%

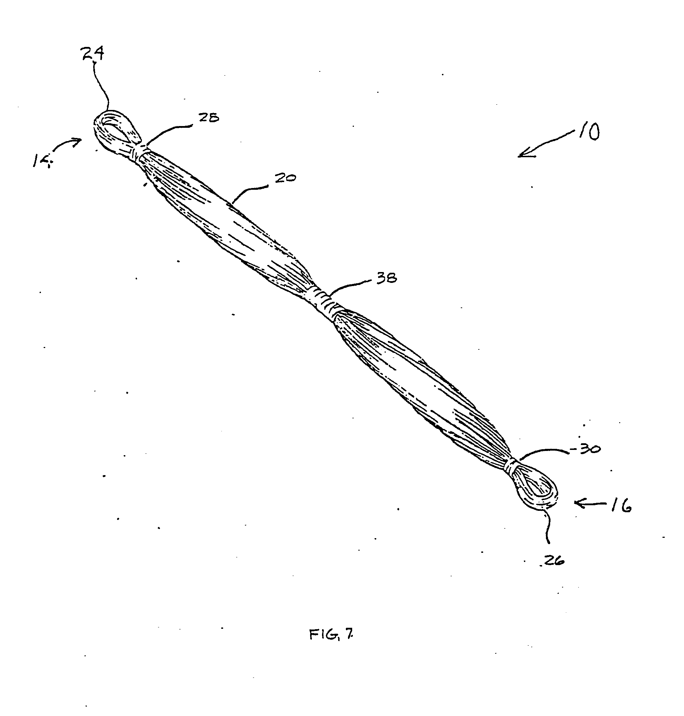

The bundle of strands was wrapped with this thin film at two points 28,30 adjacent to the spools 42,44 thereby forming eyelets 24,26 at the ends of the construction (Figure 7). A central portion 38 was also wrapped with film. The two spools were then demounted from the studs and placed on a rack constructed of thin metal wire designed to prevent rotation and longitudinal shrinkage. This rack was then exposed to 375°C in an air oven for six minutes. After cooling, the spools were demounted from the ends of the construction. The position occupied by the spools provided eyelets through which this ligament prosthesis construction can be attached to bone with screws or other suitable means of fixation. All areas which had been wrapped with film had become compressed during the heating treatment due to film shrinkage, thereby providing strand-to-strand cohesion. During the previously described heating cycle some fibre-to-fibre attachment in the unwrapped regions also took place. These fibres were then individually separated using a metal pick. The construction then comprised 160 microporous PTFE strands connecting two eyelets of somewhat densified material. Prosthesis 10 included a 180° twist along the tensile load direction to better distribute the tensile load among strands 20. PTFE tape wrap 38 surrounding strands 20 and positioned approximately midway between ends 14,16 of prosthesis 10 serves to maintain the twist by securing strands 20 against untwisting during implantation. As with PTFE wraps 28,30, wrap 38 is intended to be positioned outside of the bone contact area so as not to inhibit tissue ingrowth into strands 20.

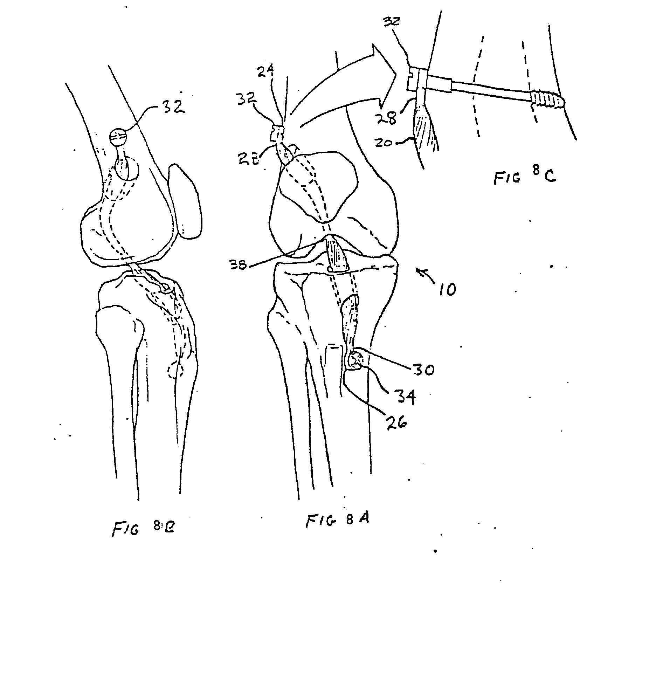

A device prepared in the manner just described was implanted into the knee of a sheep to replace the excised host anterior cruciate ligament (see Figures 8A, B and C). This implantation was accomplished through the placement of one 1/4" (0.64 cm) drill hole in both the tibia and femur. The placement of the hole in the tibia was along the axis of the previously removed natural anterior cruciate and exited at the insertion site. The placement of the femoral drill hole began at the lateral distal femoral surface proximal to the femoral epicondyle. The tunnel was angled such that the exit hole was created just proximal to the lateral femoral condyle on the popliteal surface of the femur. The prosthesis 10 was routed from the femoral exit site through the intercondylar space, across the intra-articular space, and through the tibial tunnel. The eyelets 24,26 and wrapped segments 28,30 at the end of the construction were positioned to be to the outside of the drilled bone tunnels. The placement of the wrapped segment 32,34 and 38 at the center region of the construction was in the intra-articular space. The prosthesis 10 was then anchored to bone with self-tapping orthopaedic screws placed through the eyelets 24,26. The knee joint was determined to be stable immediately after the operation.

After three months implant time, the knee was removed from the animal and drill holes placed in the tibia and femur into which clamps were mounted to provide for tensile testing along the axis of the ligament construction. After removal of muscle tissue and severing of all supporting collateral structures about the knee, the femur was distracted from the tibia at a constant rate of 500mm per minute until failure. System failure took place at 642 lb (291 kg). The failure took place in the ligament prosthesis at the eyelet secured to the femur. Rupture took place as the load exceeded the fixation provided by tissue ingrowth into the intra-osseous segments and was transferred to the fixation screw. Device failure was related to an unwinding of the strand material through the eyelet segments after several strands had failed. Histologic inspection of this sample showed tissue ingrowth among and into the strands. Tissue ingrowth had proceeded completely through the diameter of some strands. We anticipate that with longer implant times the majority of strands would have shown complete and thorough ingrowth.

Attention is directed to our divisional application EP—A—0192 949, which, inter alia, claims a method for making a tensile load-bearing tissue prosthesis having a plurality of parallel longitudinally adjacent strands positioned between and connected to at least one eyelet for initially attaching the prosthesis to tensile force-applying bone tissue, the method characterised by the steps of:

a. arranging and spacing a plurality of pin means to define an elongate area in accordance with the desired size of the prosthesis;

b. forming a plurality of elongate loops around the plurality of pin means from a continuous filament of the desired strand material until the desired number of parallel strands is obtained; and

c. gathering the loop ends at one end of the elongate area to form the eyelet, said gathering step including the step of securing the gathered loop ends against ungathering.

**Claims**

1. A tensile load-bearing tissue prosthesis for connection between tensile force-applying tissues, comprising a plurality of longitudinally adjacent, parallel, non-adhering strands of polytetrafluoroethylene, at least in the portion wherein attachment to the tensile force-applying tissue is required, characterised in that the plurality of strands are of microporous polytetrafluoroethylene material having interconnecting voids defined by nodes and fibrils, the said strands being of a type and of a thickness which permits tissue ingrowth to occur across substantially the entire strand thickness.

2. A prosthesis according to claim 1 characterised in that the characteristic interstitial dimension of the material is greater than about 7 microns and the strand thickness is determined from the following relationships:

$1n$ (strand diameter) $\leq 2.28 \times 10^{-2}$ (CID) $- 4.36$ for CID $> 7$ microns, $\leq 120$ microns

$1n$ (strand diameter) $\leq 6.98 \times 10^{-2}$ (CID $- 9.94$ for CID $\geq 120$ microns

where:
CID = characteristic interstitial dimension (microns)
1n = natural logarithm
strand diameter is expressed in inches

3. A prosthesis according to claim 1 or claim 2 characterised in that the porosity is greater than about 30%.

4. A prosthesis according to claim 3 characterised in that the matrix tensile strength of the material is greater than about 20,000 psi (138 MPa).

5. A prosthesis according to claim 3 characterised in that the matrix tensile strength of said material is greater than about 40,000 psi (276 MPa).

6. A prosthesis according to claim 4 or claim 5 characterised in that the strand thickness is less than 0.08 inch (0.2 cm), the characteristic interstitial dimension is about 74 microns, the matrix tensile strength of the material is about 74,000 psi (511 MPa), and the porosity of the material is about 81%.

7. A prosthesis according to claim 6 characterised in that the strand thickness is about 0.026 inch (0.066 cm).

8. A prosthesis according to claim 1 further characterised by means for distributing the tensile load among the individual strands when the prosthesis 15 is intended to be passed over a radius.

9. A prosthesis according to claim 8 characterised in that said load distributing means comprises said plurality of strands being twisted about the tensile 20 load direction.

10. A prosthesis according to claim 9 characterised in that the angle of twist is about 180°.

11. A prosthesis according to claim 8 characterised in that said load distributing means comprises the strands being loosely braided.

12. A prosthesis according to claim 1 characterised in that said parallel strands are formed from a plurality of elongated concentric loops, said loops being formed from continuous filament of said material, wherein said strands are gathered at least at one elongate area end to form at least one eyelet for initially attaching the prosthesis to tensile force-applying bone tissue, and wherein the prosthesis further includes means for restraining the gathered loop ends against ungathering.

13. A prosthesis according to claim 12 characterised in that two opposing eyelets are formed by the gathered loop ends.

14. A prosthesis according to claim 12 or 13 characterised in that each of said eyelets are the strand portions adhered to one another to provide uniform load sharing.

15. A method for making a tensile load-bearing tissue prosthesis according to claim 1 and having said plurality of parallel longitudinally adjacent strands positioned between and connected to at least one eyelet for initially attaching the prosthesis to tensile force-applying bone tissue, the method characterised by the steps of:

a. arranging and spacing a plurality of pin means to define an elongate area in accordance with the desired size of the prosthesis;

b. forming a plurality of elongate loops around the plurality of pin means from a continuous filament of the desired strand material until the desired number of parallel strands is obtained; and

c. gathering the loop ends at one end of the elongate area to form the eyelet, said gathering step including the step of securing the gathered loop ends against ungathering.

16. A method according to claim 15 characterised by the additional steps of imparting a twist to the loop strands about the longitudinal axis of the prosthesis to provide more even load distribution when the prosthesis is intended to be passed over a radius.

17. A method according to claim 16 characterised in that the loop strands are twisted about 180°.

18. A method according to claim 15 characterised by the further step of loosely braiding the loop-strands to provide more even load distribution when the prosthesis is intended to be passed over a radius.

19. A method according to claim 15 characterised in that the securing against ungathering step includes the step of compressing and heating the gathered ends at a temperature and for a time sufficient to coalesce the loop ends.

20. A method according to claim 15 or claim 19 characterised in that the securing against ungathering step also includes the preliminary step of wrapping strands of a high strength material about the gathered ends in a direction tangential to the prosthesis axis at a position adjacent to the eyelet.

21. A method according to claim 19 characterised in that the high strength material is expanded PTFE having a matrix tensile strength of about 70,000 psi (483 MPa).

22. A method according to claim 19 characterised in that the gathered ends are compressed and heated in a die having the desired eyelet shape and dimensions.

**Patentansprüche**

1. Eine Zugbelastung tragende Gewebsprothese zur Verbindung zwischen eine Zugkraft ausübenden Geweben umfassend eine Vielzahl von longitudinal benachbarten, parallelen, nichthaftenden Strängen von Polytetrafluoräthylen zumindest in dem Teil, in dem eine Befestigung an dem die Zugkraft ausübenden Gewebe erforderlich ist, dadurch gekennzeichnet, daß die Vielzahl von Strängen aus mikroporösem Polytetraafluoräthylenmaterial mit untereinander verbundenen Leerräumen, die von Knoten und Fibrillen definiert sind, besteht, wobei diese Stränge von einer Art und einer Dicke sind, die das Einwachsen des Gewebes über praktisch die gesamte Strangdicke ermöglichen.

2. Prothese nach Anspruch 1, dadurch gekennzeichnet, daß die charakteristische Zwischenraumdimension des Materials größer als etwa 7 Mikron ist und die Strangdicke aus den folgenden Beziehungen bestimmt wird:

1n (Strangdurchmesser) $\leq$ 2,28 x $10^{-2}$ (CID) — 4,36 für CID > 7 Mikron, $\leq$ 120 Mikron

1n (Strangdurchmesser) $\leq$ 6,98 x $10^{-2}$ (CID) — 9,94 für CID $\geq$ 120 Mikron,

worin CID = die charakteristische Zwischenraumdimension (Mikron),

1n = der natürliche Logarithmus,

wobei der Strangdurchmesser in Zoll ausgedrückt ist.

3. Prothese nach Anspruch 1 oder 2, dadurch gekennzeichnet, daß die Porosität größer als etwa 30% ist.

4. Prothese nach Anspruch 3, dadurch gekennzeichnet, daß die Matrixzugfestigkeit des Materials größber als etwa 20,000 psi (138 MPa) ist.

5. Prothese nach Anspruch 3, dadurch gekennzeichnet, daß die Matrixzugfestigkeit des genannten Materials größer als etwa 40,000 psi (276 MPa) ist.

6. Prothese nach Anspruch 4 oder 5, dadurch gekennzeichnet, daß die Strangdicke kleiner als 0,08 Zoll (0,2 cm), die charakteristische Zwischenraumdimension etwa 74 Mikron, die Matrixzufestigkeit des Materials etwa 74,000 psi (511 MPa) und die Porosität des Materials etwa 81% sind.

7. Prothese nach Anspruch 6, dadurch gekennzeichnet, daß die Strangdicke etwa 0,026 Zoll (0,066 cm) beträgt.

8. Prothese nach Anspruch 1, gekennzeichnet durch eine Einrichtung zum Verteilen der Zugbelastung unter den einzelnen Strängen, wenn die Prothese über eine Krümmung geleitet werden soll.

9. Prothese nach Anspruch 8, dadurch gekennzeichnet, daß die genannte belastungsverteilende Einrichtung die genannte Vielzahl von Strängen umfaßt, die um die Zugbelastungsrichtung verdreht sind.

10. Prothese nach Anspruch 9, dadurch gekennzeichnet, daß der Drehwinkel etwa 180° beträgt.

11. Prothese nach Anspruch 8, dadurch gekennzeichnet, daß die genannte belastungsverteilende Einrichtung Stränge umfaßt, die locker verflochten sind.

12. Prothese nach Anspruch 1, dadurch gekennzeichnet, daß die genannten parallelen Stränge aus einer Vielzahl von länglichen konzentrischen Schleifen gebildet sind, welche Schleifen aus einer kontinuierlichen Faser des genannten Materials gebildet sind, wobei die genannten Stränge zumindest an einem Ende der länglichen Fläche unter Bildung zumindest einer öse zum anfänglichen Befestigen der Prothese an dem die Zugkraft ausübenden Knochengewebe vereinigt sind, und wobei die Prothese weiter Mittel aufweist, um die vereinigten Schleifenenden an Auftrennung zu hindern.

13. Prothese nach Anspruch 12, dadurch gekennzeichnet, daß zwei gegenüberliegende ösen durch die vereinigten Schleifenenden gebildet sind.

14. Prothese nach Anspruch 12 oder 13, dadurch gekennzeichnet, daß jede der genannten ösen die Strangteile sind, die unter Vorsehen einer gleichförmigen Belastungsverteilung aneinander haften.

15. Verfahren zum Herstellen einer eine Zugbelastung tragenden Gewebsprothese nach Anspruch 1, die die genannte Vielzahl von parallelen longitudinal benachbarten Strängen aufweist, die zwischen und in Verbindung mit zumindest einer öse zum anfänglichen Befestigen der Prothese an dem die Zugkraft ausübenden Knochengewebe angeordnet sind, welches Verfahren durch die Schritte

a. des Anordnens einer Vielzahl von Dornen in einem Abstand, um eine längliche Fläche entsprechend der gewünschten Größe der Prothese zu definieren,

b. des Bildens einer Vielzahl von länglichen Schleifen rund um die Vielzahl der Dorne aus einer kontinuierlichen Faser des gewünschten Strangmaterials, bis die gewünschte Zahl von parallellen Strängen erzielt ist, und

c. des Vereinigens der Schleifenenden an einem Ende der länglichen Fläche unter Bildung der öse, wobei der genannte Vereinigungsschritt das Sichern der vereinigten Schleifenenden gegen Auftrennung einschließt, gekennzeichnet ist.

16. Verfahren nach Anspruch 15, gekennzeichnet durch den weiteren Schritt, daß den Schleifensträngen um die Längsachse der Prothese eine Drehung erteilt wird, um eine gleichmäßigere Belastungverteilung vorzusehen, wenn die Prothese über eine Krümmung geleitet werden soll.

17. Verfahren nach Anspruch 16, dadurch gekennzeichnet, daß die Schleifenstränge um etwa 180° verdreht werden.

18. Verfahren nach Anspruch 15, gekennzeichnet durch den weiteren Schritt des lockeren Verflechtens der Schleifenstränge, um eine gleichmäßigere Belastungsverteilung vorzusehen, wenn die Prothese über eine Krümmung geleitet werden soll.

19. Verfahren nach Anspruch 15, dadurch gekennzeichnet, daß der Schritt des Sicherns gegen Auftrennung das Komprimieren und Erhitzen der vereinigten Enden auf eine ausreichende Temperatur und während eines ausreichenden Zeitraumes, um die Schleifenenden zu verschmelzen, einschließt.

20. Verfahren nach Anspruch 15 order 19, dadurch gekennzeichnet, daß der Schritt des Sicherns gegen Auftrennung auch das vorhergehende Umwickeln von Strängen aus Material mit hoher Festigkeit um die vereinigten Enden in einer Richtung tangential zur Prothesenachse an einer der öse benachbarten Stelle einschließt.

21. Verfahren nach Anspruch 19, dadurch gekennzeichnet, daß das Material mit hoher Festigkeit expandiertes PTFE mit einer Matrixzugfestigkeit von etwa 70,000 psi (483 MPa) ist.

22. Vefahren nach Anspruch 19, dadurch gekennzeichnet, daß die vereinigten Enden in einer Form mit der gewünschten ösengestalt und den gewünschten Dimensionen komprimiert und erhitzt werden.

## Revendications

1. Prothèse de tissu supportant un effort de traction destinée à la connexion entre des tissus appliquant une force de traction, comprenant une série de fils de polytétrafluoroéthylène non adhérents, parallèles, longitudinalement adjacents, au moins dans la partie dans laquelle une fixation au tissu appliquant une force de traction est requise, caractérisée en ce que la série de fils sont en une matière de polytétrafluoroéthylène microporeuse, comportant des vides d'interconnexion définis par des noeuds et des fibrilles, ces fils étant d'un type et d'une épaisseur qui permettent la croissance intérieure de tissu sur pratiquement l'épaisseur de fil totale.

2. Prothèse suivant la revendication 1, caractérisée en ce que la dimension interstitielle caractéristique de la matière est supérieure à environ 7 microns et l'épaisseur de fil est déterminiée d'après les relations suivantes:
1n (diamètre de fil) $\leq 2,28 \times 10^{-2}$ (CID) — 4,36 pour CID > 7 microns, $\leq 120$ microns
1n (diamètre de fil) $\leq 6,98 \times 10^{-2}$ (CID) — 9,94 pour CID $\geq 120$ microns
où:
CID = dimension interstitielle caractéristique (microns)
1n = logarithme naturel
le diamètre des fils est exprimé en pouces.

3. Prothèse suivant l'une ou l'autre des revendications 1 et 2, caractérisée en ce que la porosité est supérieure à environ 30%.

4. Prothèse suivant la revendication 3, caractérisée en ce que la résistance à la traction matricielle de la matière est supérieure à environ 20,000 psi (138 MPa).

5. Prothèse suivant la revendication 3, caractérisée en ce que la résistance à la traction matricielle de la matière est supérieure à environ 40,000 psi (276 MPa).

6. Prothèse suivant l'une ou l'autre des revendications 4 et 5, caractérisée en ce que l'epaisseur de fil est inférieure à 0,08 pouce (0,2 cm), la dimension interstitielle caractéristique est d'environ 74 microns, la résistance à la traction matricielle de la matière est d'environ 74,000 psi (511 MPa) et la porosité de la matière est d'environ 81%.

7. Prothèse suivant la revendication 6, caractérisée en ce que l'épaisseur de fil est d'environ 0,026 pouce (0,066 cm).

8. Prothèse suivant la revendication 1, caractérisée en outre par un moyen pour distribuer l'effort de traction parmi les fils individuels lorsque la prothèse est utilisée pour être passée au-dessus d'un arrondi.

9. Prothèse suivant la revendication 8, caractérisée en ce que le moyen de distribution d'effort comprend la série de fils précitée en torsion autour de la direction d'effort de traction.

10. Prothèse suivant la revendication 9, caractérisée en ce que l'angle de torsion est d'environ 180°.

11. Prothèse suivant la revendication 8, caractérisée en ce que le moyen de distribution d'effort précité comprend les fils entrelacés de façon lâche.

12. Prothèse suivant la revendication 1, caractérisée en ce que les fils parallèles précités sont formés à partir d'une série de boucles concentriques allongées, ces boucles, étant formées à partir d'un filament continu de la matière précitée, les fils étant réunis au moins en une extrémité de zone allongée pour former au moins un oeillet pour fixer initialement la prothèse à du tissu osseux appliquant une force de traction, la prothèse comprenant en outre un moyen pour empêcher les extrémités bouclées réunies de se désunir.

13. Prothèse suivant la revendication 12, caractérisée en ce que deux oeillets opposés sont formés par les extrémités bouclées réunies.

14. Prothèse suivant l'une ou l'autre des revendications 12 et 13, caractérisée en ce que chacun des oeillets précités est formé des portions de fils adhérant mutuellement pour procurer une répartition d'effort uniforme.

15. Procédé de fabrication d'une prothèse de tissu supportant un effort de traction suivant la revendication I et comportant la série précitée de fils adjacents, longitudinalement parallèles, positionnés entre et reliés à au moins un oeillet pour fixer initialement la prothèse à du tissu osseux appliquant une force de traction, ce procédé étant caractérisé par les étapes suivantes:
a) l'agencement et l'espàcement d'une série de moyens de broche pour définir une zone allongée suivant la taille désirée de la prothèse;
b) la formation d'une série de boucles allongées autour de la sefie de moyens de broche à partir d'un filament continu de la matière de fil désirée jusqu'à ce que le nombre désiré de fils parallèles soit obtenu; et
c) la réuniton des extrémités bouclées à une extrémité de la zone allongée pour former l'oeillet, cette étape de réunion comprenant l'étape permettant d'empêcher les extrémités bouclées réunies de se désunir.

16. Procédé suivant la revendication 15, caractérisé par les étapes additionnelles permettant de conférer une torsion aux fils bouclés autour de l'axe longitudinal de la prothèse pour obtenir une distribution d'effort plus régulière lorsque la prothèse est utilisée pour être passée au-dessus d'un arrondi.

17. Procédé suivant la revendication 16, caractérisé en ce que les fils bouclés sont tordus à environ 180°.

18. Procédé suivant la revendication 15, caractérisé par l'étape supplémentaire d'enchevêtrement lâche des fils bouclés pour obtenir une distribution d'effort plus régulière lorsque la prothèse est utilisée pour être passée au-dessus d'un arrondi.

19. Procédé suivant la revendication 15, caractérisé en ce que l'étape permettant d'empêcher la désunion comprend l'étape de compression et de chauffage des extrémités réunies à une température et pendant un temps suffisants pour associer les extrémités bouclées.

20. Procédé suivant l'une ou l'autre des revendications 15 et 19, caractérisé en ce que l'étape permettant d'empêcher la désunion comprend également l'étape d'enveloppement préliminaire des fils d'une matière de résistance élevée autour des extrémités réunies dans une direction tangentielle à l'axe de la prothèse en une position adjacente à l'oeillet.

21. Procédé suivant la revendication 19, caractérisé en ce que la matière de résistance élevée est du PTFE expansé ayant une résistance à la traction matricielle d'environ 70,000 psi (483 MPa).

22. Procédé suivant la revendication 19, caractérisé en ce que les extrémités réunies sont comprimées et chauffées dans une matrice ayant es dimensions et la forme d'oeillet désirées.

FIG. 1

FIG. 2

FIG. 3

FIG. 4

FIG. 5

FIG. 6

FIG. 7

FIG 8 C

FIG 8 B

FIG 8 A